# EUROPEAN PATENT APPLICATION

(11) **EP 2 157 187 A2**
(43) Date of publication of application: **24.02.2010**
(21) Application number: 08794074.8
(22) Date of filing: 11.07.2008
(51) Int. Cl.: C12P 19/34, C12N 15/10, C12M 1/38

(54) **CONVECTION POLYMERASE CHAIN REACTION METHOD**

(30) Priority: 14.06.2007 RU 2007121893
(71) Applicant: Institut Biokhimii I Genetiki Ufimskogo Nauchnogo Tsentra Ran, Ufa 450054 (RU)
(72) Inventor: CHEMERIS, Dmitry Alekseevich, Ufa, 450097 (RU); CHEMERIS, Aleksei Viktorovich, Ufa, 450097 (RU); MAGDANOV, Eduard Gavisovich, Ufa, 450077 (RU); GARAFUTDINOV, Ravil Rinatovich, Meleuz, 453310 (RU); VAKHITOV, Vener Absatarovich, Ufa, 450006 (RU); URMANCHEEV, Said Fedorovich, Ufa, 450040 (RU); LEBEDEV, Yury Anatolievich, Ufa, 450075 (RU)
(74) Representative: Henriksson, Dan Ragnar Mikael
(86) International application number: PCT/RU2008/000462
(87) International publication number: WO 2008/153447

(57) **Abstract**

The invention relates to a method for rapid detection of specific nucleic acid fragments with the aid of a PCR, consisting in amplifying target products in a special DNA thermocycler, provided with a special reaction thermal unit, which makes it possible to obtain, in reaction vessels in the form of standard polypropylene test tubes, a sloping temperature gradient directed at an angle to the direction of gravity. The amplification time is of 1-5 minutes. The inventive method can be recommended for DNA diagnostics, also field conditions, in medicine, veterinary sciences, in sanitary and epidemiological studies for detecting agents of dangerous infections, including potential bio-terrorist attacks, in criminalistics for identifying criminals in the food industry for detecting food products from genetically modified organisms, for testing raw material quality etc.

## Description

The invention relates to molecular biology and biotechnology and is linked to an analysis of nucleic acid molecules by amplification of specific fragments thereof. It may be used for DNA diagnostics, also in field conditions, in medicine, veterinary, in sanitary and epidemiological studies for detecting agents of dangerous infections, including potential bio-terrorist attacks, in criminalistics for identifying criminals, in the food industry for detecting food products from genetically modified organisms, for determining raw material quality, etc.

Existing methods for DNA diagnosis are mainly based on amplification of specific DNA or RNA fragments with the aid of a polymerase chain reaction (PCR) and modifications thereof. In recent years, in addition to the usual PCR at the final point, which requires electrophoretic separation of the reaction products (or the analysis thereof by some other method), the so-called real time PCR is being used more and more, when detection of the target product is carried out directly during amplification with the aid of special DNA thermocyclers equipped with an optical module.

After it appeared in the middle of the eighties in the twentieth century, PCR, at least in respect to the scale of its use, rather rapidly became the actual method No. 1 in the biomedical sciences and in diagnostics. Nevertheless, following PCR, mainly at the turn of the nineties, a large number of other amplification reactions and methods of highly sensitive detection of specific DNA or RNA fragments appeared. However, not a single one of those methods or methods appearing later, right up to the present time, could for a number of reasons become a serious rival of PCR.

In order to carry out PCR, a cyclic change of temperature is necessary since in order for a new cycle to begin, denaturation of amplicons should take place (usually at a temperature of about 95°C) with the subsequent annealing of primers (most often at temperatures from 50 to 60°C) and their elongation (temperatures of 72-75°C are most often used). But in view of the fact that the temperature transitions in the reaction unit and accordingly in the reaction mixture do not take place instantaneously, a significant time is spent on heating and cooling the unit itself and then the content of the test-tubes to the necessary temperatures, which often requires more than 80% of the total time of the reaction. In order to accelerate the change of the temperature in the reaction mixture itself in the PCR, it is proposed that special thin-wall polypropylene test tubes, which are at present supplied by many firms, be used. Nevertheless, virtually right after the appearance of the PCR method, the development began of all kinds of methods for rapidly changing the temperature in reaction test-tubes. So, in the first commercial models of thermocyclers, heating the reaction units was carried out by electrical heaters, while cooling was with either air or water. At the present time the great majority of thermocyclers are based on Peltier elements, which make it possible to rather easily and rapidly change the temperature in the reaction unit. The speed of temperature change of different models of thermocyclers based on Peltier elements of different firms is on the average from 2 to 6°C per second. Wherein, the reaction units of some high-speed thermocyclers are not produced from usually used aluminum, but from gold-plated silver, which significantly better conducts heat, but is significantly more expensive. As a rule, the amplification of DNA fragments of a size up to 1000 pairs of nucleotides during 25-30 cycles in thermocyclers of such a type takes from 30 minutes to 2 hours.

An alternative to thermocyclers based on Peltier elements is provided by devices, wherein very rapid heating (to 15°C per second) is carried out by a powerful infrared lamp, cooling - by a powerful ventilator [Wittwer et al., 1990; 1995]. In that case, a reaction unit as such is missing, and instead of it there is an air chamber, which removes the inertia of the temperature change, since air of one temperature is very rapidly replaced by air of another, as a result of which the typical 30 cycles in such devices are completed in 15 minutes. However, the fact that the reaction, as a rule, is carried out in special glass capillaries, not in standard polypropylene test-tubes, which are significantly cheaper and more convenient, is a drawback.

A more rapid change of the temperature in the reaction unit is achieved by alternately feeding hot and cold gases under pressure, which however, makes the whole construction significantly more complex. So, one of the most rapid DNA thermocyclers at the end point, that is working due to the action of the gases, received the name PCRJet. It is indicated that with the use thereof, 35 cycles are carried out in 8 minutes [Ebmeier et al., 2004]. In the US patent for a thermocycler, which is based on the same principle, consideration is given to a temperature change at a rate of 17°C per second [Quintanar, Nelson, 2002]. The use of special cuvettes instead of standard convenient test-tubes is a further drawback of these models in addition to the necessity to use gases under pressure.

Another solution in respect to the rapid temperature change is realized in Robocycler devices from Stratagene, in which there are 4 units, each constantly maintaining its temperature (or the temperature gradient in some models), between which mechanical displacement of the reaction plates takes place with the use of a manipulator, for which time is also required. Therefore, the length of only one cycle is about 3 minutes.

All of the aforesaid methods for a rapid change of the temperatures also presume that a uniform temperature will be provided inside the air chamber or metal thermal unit that differs in respect to test tubes at different locations by tenths of a degree.

An approach that is different in principle is the method of carrying out PCR in a thermocycler of original construction (Nakano et al., 1994). The main specificity of this device was that the polymerase chain reaction did not take place as usual in a test tube, but in a flow of liquid, constantly flowing with the aid of a special pneumatic pump along a teflon capillary, separate sections of which were arranged in zones with corresponding temperatures of DNA denaturation, annealing of the primers and elongation of new chains. So, definite parts of the general reaction mixture in that case are simultaneously in different temperature conditions, which cardinally distinguishes this regimen as compared with classical approaches and has some advantages, as a result of which this idea subsequently received significant development upon the development of PCR in microfluid devices.

A constantly changing nonuniform temperature in a reaction mixture is also customary for the cases of carrying out PCR with use of the thermal convection effect. In one variant of the convection PCR, a change of the temperature inside the reaction mixture takes place due to the gradient of the surface tension force caused by the temperature differences, and such a convection cell has the name Marangoni cell. Proposed on the basis thereof is a method of carrying out PCR, which is designed on carrying out amplification in a flowing microfluid device, not in test tubes [Lee et al., 2006]. The low speed at which the reaction takes place and poor scalability are drawbacks thereof.

Another mechanism for moving the layers of liquid, which lies at the basis of the so-called Rayleigh-Benard convection cell, is brought into action by the buoyancy force, which is the difference between the Archimedean force and the gravity force. The convection cell for carrying out PCR was proposed on this principle, this cell being a vertical channel with a depth of 1.5 cm and volume of 35 µl in a cube of organic glass [Krishnan et al., 2002]. The cube was heated from the bottom to 97°C, from the top it was thermostat controlled at a temperature of 61°C. As a result a specific PCR product was produced in 1.5 hours of the reaction, the amount of which product turned out to be sufficient for it to be seen with use of agarose gel electrophoresis.

Later, the convection PCR system was somewhat improved by the same authors [Krishnan et al., 2004], but the main drawbacks in the form of a low reaction speed and difficulty in filling the reaction vessels remained. So, instead of a single channel cube, a special multiwell cartridge was produced from organic glass, which at the top and bottom was heated to the necessary temperatures by aluminum plates. However, it may be stated that the serious problem of eliminating the formation of air bubbles at the top of the cavity, which are capable of significantly changing the established temperature regimen, has not been resolved, since it was proposed to slightly overfill the cavity with liquid, without thereby allowing cross contamination of the samples, which would immediately show the absolute non-technological character of this method of amplification. Removal of the liquid from these vertical, extremely narrow channels after completion of the reaction is difficult or, at least, rather lengthy. Another variant of the reaction vessels, also proposed in this paper, were 9 cm piece of a polymer tubing, which contained 15 µl of liquid. As in the case with a cartridge with wells, an overfill was also recommended upon filling, which should be carried out by bending the tubing in the form of the letter U. In that case the presence of a small air bubble is not that dangerous for conduction of the reaction. Closing the tubing into a ring after it is filled is carried out by a small piece of another tubing of a suitable diameter, after which such a reaction vessel may acquire different forms, providing for the application of necessary temperatures to corresponding sections of the tubing, for the beginning of convection cells and flows of liquid. It is indicated that in such a convection PCR about 40 minutes of incubation are required for the production of the target product, which in respect to amount is comparable with the same upon carrying out the usual PCR [Krishnan et al., 2004]. The method for carrying out PCR in a piece of a tubing was later further developed, which made it possible for the same authors [Agrawal, Ugaz, 2007; Agrawal et al., 2007] to create a pocket convection thermocycler, powered by type AA batteries. But wherein, such a portable method of amplification with the aid of convection PCR retained all of the drawbacks of the larger-size previous construct.

One more type of convection cell for carrying out PCR was proposed by other authors [Braun et al., 2003]. In spite of the fact that they were able to achieve a relatively high speed of amplification, completed in about 10 minutes, due to the fact that the time spent on one standard cycle (denaturation, annealing, elongation) was only about 15 seconds, their reaction vessel was a thin layer of liquid between the cover glasses for microscopic studies, sealed with silicon, and cannot in any way be recommended for wide experiments and even more so for DNA diagnostics.

A cell of even more complicated structure with a vertical temperature gradient, in which convection should arise and PCR take place, is proposed in patent US No. 6,586,233 [Bennet et al., 2003]. However, the massive scale of use of this method for convection PCR is extremely doubtful in view of the complication and the evident expensiveness of the working chamber itself, where amplification takes place. In the work of other authors [Hwang et al., 2004], a thick-walled glass tube that was open at the upper end served as the reaction vessel. Its height was 55-60 mm, inner diameter - 2 mm, outer diameter - 8 mm, the lower end had a wall thickness of 3 mm. It was placed in a solid-body reaction block, separated by an isolator into an upper part and a lower part, each being given its own temperature. The target product appeared for electrophoresis only after a one-hour incubation, wherein during the convection PCR the reaction vessel was under a pressure of nitrogen equal to 1.2 atmospheres. All of these complexities also raise doubts in respect to the large-scale use of such a convection PCR.

So, not a single one of the methods of convection PCR that are proposed at present provides for a really rapid temperature change or is intended for wide use.

The prototypes that are most similar to the method of amplifying nucleic acids with use of convection PCR that we have proposed are methods that are based on Rayleigh-Benard convection cells, since the contribution of surface tension forces that are typical for Marangoni cells in our case is not essential.

The object of the invention is to significantly accelerate the conduction of PCR with use of convection, based on the buoyancy force, and to simplify that process with the retention of high specificity of the reaction.

The essence of the invention consists in that rapid amplification of the target products of the PCR is carried out in a special DNA thermal cycler provided with a specific reaction thermal block providing in reaction vessels, which are standard polypropylene test tubes, a sloping gradient of temperature, oriented at an angle to the direction of action of the force of gravity.

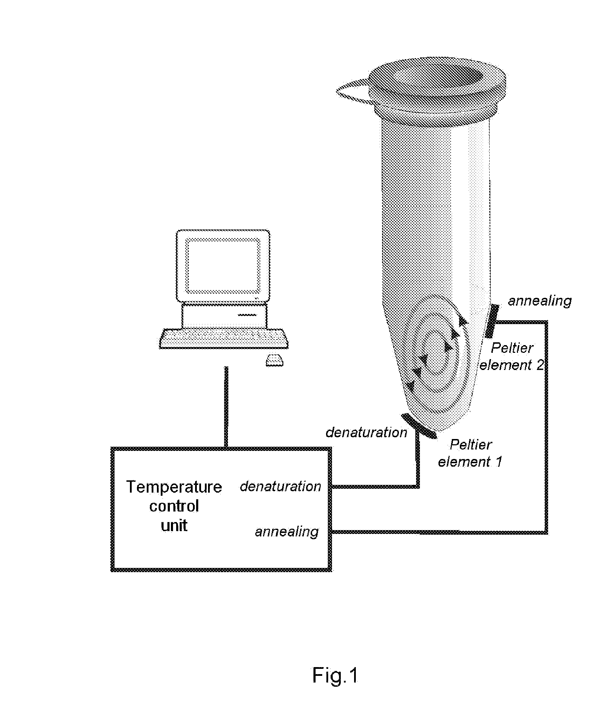

The reaction block in the convection DNA thermocycler is provided by two aluminum (or from another suitable metal) bands with special cavities (for the lower) and curvatures (for the upper or side bands), providing maximum close contact with the necessary places of the reaction polypropylene 0.2 ml test tubes usually containing 30 µl of the reaction mixture (Fig. 1). The retention of the required temperature of the metal bands is carried out with the aid of Peltier elements and is controlled by a processor. In view of the short period of conduction of the reaction (1-5 minutes) prevention of evaporation is not required with use of either a hot lid or layering mineral oil.

In another variant, the lower temperature (denaturation temperature) is maintained not by an aluminum strip, but rather with use of a metal (aluminum) plate with corresponding multiple cavities, the number of which coincides with the number of used test tubes and is the capacity of the thermocycler. The form of the cavities repeats the profile of the close-to-bottom part of the test tube so that a close contact is only provided with one side of the bottom and a side. In view of the standard square cluster positioning of the test tubes, this reaction unit does not require alteration of the standard optical modules for registration of the course of the reaction in the real time mode.

After the predetermined temperatures are applied to the reaction test tubes, a sloping temperature gradient is very rapidly created inside them, which initiates the convection mechanism that is based on the force of buoyancy. Wherein, due to the slope temperature gradient, a directed movement of the layers of liquid occurs, which do not break up into multiple Benard cells as often takes place in the case when a vertical temperature gradient is applied. The speed of rotation of the liquid along an ellipse-like path, which was from 2 to 3 seconds per revolution, was assessed with the aid of special sampling particles. Due to this, the liquid in that period of time passes from the high temperature zone (the denaturation zone) through the medium temperature zone (inactive area without DNA annealing and polymerization), enters the low temperature zone (annealing zone), again enters the medium temperature zone (elongation zone) and then again the high temperature zone etc. So, one cycle takes about 2-3 seconds and for 1-2 minutes there are from 20 to 60 cycles. In view of the fact that the liquid in the middle part of the test tube traverses a smaller ellipse, the DNA molecules inside it do not turn out to be within the high temperature zone where denaturation of the amplicons could take place and then accordingly annealing of the primers, which accompanies the elongating thereof. However, due to the diffusion that is present and the fluctuations of the flows, the layers of liquid from the central part mix and also turn out to be involved in that temperature cycle. Wherein, it does not make any difference that all of the amplicons do not simultaneously conduct themselves in the same manner. What is important is that the molecules could multiply in time to such a degree that this could be registered either at the end point by electrophoresis or in real time by the fluorescence of a corresponding dye(s).

As regards the small amount of time in which the DNA molecules are in each of the temperature zones, it should be noted that the main intended purpose of convection PCR is not to produce long DNA fragments for their subsequent cloning or sequencing, but the purpose is to carry out mass analyses, where the size of the amplicons is usually not large and the only thing necessary is a large carrying capacity of the method when diagnostic tests are carried out. So, in order to carry out specific PCR, it is quite sufficient to detect a fragment having a size of about 40 nucleotides pairs with the aid of primers, annealing end to end (different chains) or even with conditional covering (overlapping) of 1 nucleotide. Wherein the specificity of amplification in the case of correct selection of the primers will be provided for, since the number of all the variants of the overshoot of nitrogenous bases in a fragment having a length of 40 nucleotides will have a gigantic value of 4⁴⁰, which is approximately equal to a heptillion (10²⁴) of the combination and a random location of such nucleotide sequences is possible with a probability of 8x10⁻²⁵, and since genomes with such sizes do not exist, then, consequently, such an event may be considered to be extremely improbable.

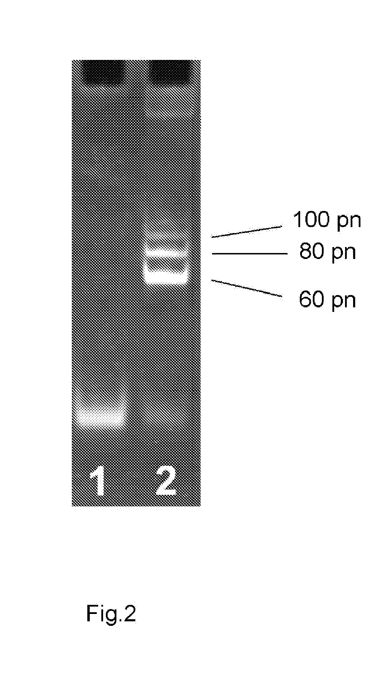

The speed of action of many thermally stable DNA polymerases is very high and for that moment (less than a second) that the amplicon with the annealed primer is in the zone that is optimum for elongation of the DNA chain, the enzyme is quite able to amplify 20 and more nucleotides. As can be seen in Fig. 2, it is quite possible with the aid of convection PCR with a sloping temperature gradient to reliably amplify DNA fragments with a length of at least to 100 nucleotide pairs. The time of annealing primers and denaturation of DNA as to the formation and destruction of hydrogen bonds, measured according to different sources from picoseconds to milliseconds, is not a limiting factor either.

The proposed method of amplifying specific DNA or RNA fragments with a polymerase chain reaction, controlled by thermal convection, is illustrated by the following examples.

### Brief description of the drawings

Fig. 1 shows a diagram of application of heating (cooling) points, providing for the initiation of a sloping temperature gradient oriented at an angle to the direction of action of the force of gravity.
Fig. 2 shows an electrophoretic analysis of the products of convection PCR with a sloping temperature gradient in an 8% polyacrylamide gel, amplified with the use of thermostable Vent exo⁻ DNA polymerase. 1 - primers; 2 - PCR products of different size, beginning with 60 nucleotide pairs with an increase of 20 nucleotide pairs (seen are free primers).

### Example 1

### Conduction of convection PCR

PCR was carried out in 30 µl of a reaction mixture containing a buffer (40 mM Tris-HCl pH 8.0. 0.25 mM MgCl₂, 25 mM KCl); 1 unit of activity Taq DNA polymerase; 0.5 pmol for each of the 2 primers and a corresponding amount of distilled water. PCR was carried out in an experimental sample of a convection DNA thermocycler, wherein in the lower part of the test tube a temperature of 95°C was retained and in the location positioned obliquely in respect to the first a temperature was set that is optimum for annealing the selected primers. Application of denaturation and annealing temperatures to the indicated places (Fig. 1) provided for the formation of a sloping temperature gradient and the initiation of convection. The time of incubation varied from 1 minute to 5 minutes.

### Example 2

### Electrophoretic analysis of PCR products

The electrophoretic separation of PCR products was carried out in 8% polyacrylamide gel in a tris-acetate buffer pH 7.8 in undenaturating conditions at a voltage gradient of 4V per cm length of the gel in a vertical type device for 4 hours. After completion of electrophoresis, the gel after staining with ethidium bromide was photographed in the Gel Camera System (UVP, Inc.) photo documention system.

### Literature

Agrawal N., Hassan Y.A., Ugaz V.M. A Pocket-Sized Convective PCR Thermocycler //Angew. Chem.. Int. Ed. Engl. 2007. V.46. P.4316-4319.
Agrawal N., Ugaz V.M. A buoyancy-driven compact thermocycler for rapid PCR // Clin. Lab. Med. 2007. V.27. P.215-223.
Bennet W.J., Richards J.B., Milanovich F.P. Convectively driven PCR thermalcycling //US Patent No. 6,586,233 B2. Jul. 1, 2003.
Braun D., Goddard N.I., Libchaber A. Exponential DNA replication by laminar convection // Phys. Rev. Lett. 2003. V.91. 158103.
Ebmeier R., Whitney S., Alugupally S., Nelson M., Padhye N., Gogos G., Viljoen H.J. Ranque - Hilsch vortex tube thermocycler for DNA amplification // Instrum. Sci. Technol. 2004. V.32. P.567-570.
Hwang H.J., Kim J.H., Jeong K. Method and apparatus for amplification of nucleic acid sequences by using thermal convection // US Patent Application Publication No. 2004/0152122 A1. Aug. 5, 2004.
Krishnan M., Agrawal N., Bums M.A., Ugaz V.M. Reactions and fluidics in miniaturized natural convection systems // Anal. Chem. 2004. V.76. P.6254-6265.
Krishnan M., Ugaz V.M., Bums M.A. PCR in a Rayleigh-Benard convection cell //Science. 2002. V.298. P.793.
Lee Y-S., Kuk K., Oh Y-S., Shih S-H., Kim M-S. Polymer chain reaction apparatus using Marangoni convection and polymer chain reaction method using the same // US Patent Application Publication No. 2006/0216725 A1. Sep. 28, 2006.
Nakano H., Matsuda K., Yohda M., Nagamune T., Endo I., Yamane T. High speed polymerase chain reaction in constant flow // Biosci. Biotech. Biochem. 1994. V.58. P.349-352.
Quintanar A., Nelson R.M. High speed process and apparatus for amplifying DNA // US Patent No. 6,472,186 B1. Oct. 29, 2002.
Wittwer C.T., Fillmore G.C., Garling D.J. Minimizing the time required for DNA amplification by efficient heat transfer to small samples // Anal. Biochem. 1990. V.186. P.328-331.
Wittwer C.T., Hillyard D.R., Ririe K.M. Rapid thermal cycling device // US Patent No. 5,455,175. Oct. 3, 1995.

## Claims

1. A method for amplification of specific fragments of nucleic acids with the aid of a polymerase chain reaction, **characterized in that** due to the effect of accelerated convection occurring as a result of a force of buoyancy caused by a sloping temperature gradient applied to a reaction vessel and oriented at an angle to the direction of action of gravity, cyclic temperature changes that are necessary for multiple sequential implementation of steps of denaturation, annealing and elongation in the reaction mixture due to constant, directed displacement of liquid layers very rapidly occur.

2. The method according to claim 1, **characterized in that** a reaction vessel is used that is a standard polypropylene test tube.

3. The method according to claim 1, **characterized in that** registration of the process of amplification of nucleic acids is carried out in the real time mode.

4. The method according to claim 1, **characterized in that** the application of identical temperatures to corresponding parts of the reaction vessel and the synchronous change thereof instead of the initiation of a temperature gradient provides sufficient homogeneity of the temperature for the whole liquid and if necessary makes it possible to carry out the usual PCR.
